Europäisches Patentamt

European Patent Office

Office européen des brevets

(19)

(11) Veröffentlichungsnummer: **0 214 486**
**B1**

# (12) EUROPÄISCHE PATENTSCHRIFT

(45) Veröffentlichungstag der Patentschrift: **31.10.90**

(21) Anmeldenummer: **86111080.7**

(22) Anmeldetag: **11.08.86**

(51) Int. Cl.⁵: **A 61 B 6/14**

(54) Zahnärztliches Röntgendiagnostikgerät.

(30) Priorität: **23.08.85 DE 3530234**

(43) Veröffentlichungstag der Anmeldung:
**18.03.87 Patentblatt 87/12**

(45) Bekanntmachung des Hinweises auf die
Patenterteilung:
**31.10.90 Patentblatt 90/44**

(84) Benannte Vertragsstaaten:
**DE FR GB IT**

(56) Entgegenhaltungen:
**EP-A-0 002 706**
**EP-A-0 193 650**
**DE-B-1 130 687**
**DE-C- 898 793**
**US-A-2 720 708**
**US-A-3 539 805**
**US-A-3 930 507**

(73) Patentinhaber: **Siemens Aktiengesellschaft**
**Wittelsbacherplatz 2**
**D-8000 München 2 (DE)**

(72) Erfinder: **Molitor, Dieter**
**Kurt-Schumacher-Strasse 9**
**D-6842 Bürstadt (DE)**
Erfinder: **Günther, Werner**
**Odenwaldstrasse 20**
**D-6140 Bensheim (DE)**

Courier Press, Leamington Spa, England.

EP 0 214 486 B1

**Beschreibung**

Die Erfindung bezieht sich auf ein zahnärztliches Röntgendiagnostikgerät zur Erstellung kongruenter Panorama-Schichtaufnahmen vom Kiefer eines Patienten, enthaltend eine Strahlenquelle und Filmkassette aufnehmende Dreheinheit und wenigstens zwei, den Patientenkopf in bezug auf die Dreheinheit festlegende positionierglieder, von denen das eine eine in der Horizontalen verstellbare Stirnstütze ist, sowie Anzeigemittel für zumindest die Verstellposition der Stirnstütze.

Bei einem bekannten solchen Röntgendiagnostikgerät (Prospekt ORTHOPANTOMOGRAPH 10 M - D 8071361; WS 08832) sind zur Positionierung des Patientenkopfes einerseits eine Stirnstütze und andererseits eine Kinnauflage mit Aufbißteil vorgesehen. Kinnauflage und Stirnstütze sind in Richtung auf das Säulenstativ, an dem die Dreheinheit mit Röntgenstrahlquelle und Filmkassette schwenkbar gehaltert ist, in der Horizontalen verstellbar. Um den Patientenkopf in bezug auf Mittigkeit und Neigung (Frankfurter Horizontale) richtig positionieren zu können, ist ferner ein Lichtvisier vorgesehen, mit dem einerseits die richtige Schädelneigung und andererseits die Gesichtsmitte eingestellt bzw. kontrolliert werden können. Die eingestellte Patientenposition ist auf einer Digitalanzeige ablesbar.

Mit einem solchen Gerät lassen sich sowohl sogenannte Standard-Aufnahmen, d.h. Aufnahmen des Unter- und Ober kiefers, als auch sogenannte Sinus-Aufnahmen, d.h. Aufnahmen der Oberkieferhöhlen sowie Kiefergelenkaufnahmen, erstellen. Die verstellbar angeordnete Kinnstütze ist mit einem verschwenkbaren Haltestäbchen mit Aufbißteil zur Fixierung der Frontzähne des Kiefers versehen. Wenngleich es mit dem beschriebenen Gerät möglich ist, reproduzierbare Aufnahmen zu erstellen, indem die Werte der Stirnstützenposition und die der Position der Kinnstütze notiert werden und diese Werte bei einer erneuten Aufnahme desselben Patienten dann wieder eingestellt werden, so entspricht die damit erzielbare Einstellgenauigkeit noch nicht der heute gestellten Forderung nach einer Abweichung der Repro-Aufnahme gegenüber der Erstaufnahme von weniger als 1 mm.

Derart genaue Repro-Aufnahmen lassen sich auch mit anderen, aus der Patentliteratur bekannt gewordenen Geräten (US-27 20 708 und DE-898 793) nicht erzielen. Diesen haftet noch der weitere Nachteil an, daß das Einstellen des Patientenkopfes vergleichsweise zeitaufwendig und umständlich ist. So müssen bei dem Gerät nach der DE-898 793 vier Positionierglieder justiert werden. Das Gerät enthält einen ringförmigen Bügel, auf dem zwei in die Öffnungen der Gehörgänge einführbare Stifte und ein an der Übergangsstelle von Oberlippe und unterer Wurzel der Nasenscheidewand anlegbares Schiebeteil verstellbar gehaltert sind. Ferner ist am Bügel noch ein Anzeigestift gehalten, mit dem die Eintrittsstelle des Röntgenstrahles mittels eines Farbtupfens auf der Haut des Patienten markiert werden kann. Der Bügel und die vier Positionierglieder bzw. deren Halter sind mit Skalen in Form von Strichmarkierungen versehen, die nach Einstellen des Patientenkopfes abgelesen und notiert werden müssen.

Bei dem Gerät nach US-PS 27 20 708, welches nicht dazu dient, Panorama-Schichtaufnahmen vom Kiefer eines Patienten machen zu können, sind Positionierglieder in Form von einerseits einem Aufbißteil für die Frontzähne und andererseits einer auf den Patientenkopf aufsetzbaren Kappe vorgesehen, die in mehrere Ebenen verstellbar aufgehängt ist, wobei die Verstellorgane jeweils mit Skalierungen versehen sind.

Die beiden bekannten Geräte bieten keine Möglichkeit, die eingangs erwähnten unterschiedlichen Aufnahmearten (Unter-, Oberkiefer, Sinus- und Kiefergelenkaufnahmen) durchzuführen, und zwar weder für einen bezahnten noch für einen unbezahnten Patienten.

Der im Anspruch 1 angegebenen Erfindung liegt die Aufgabe zugrunde, ein Röntgendiagnostikgerät der eingangs genannten Gattung dahingehend zu verbessern, daß sich mit relativ einfacher Einstelltechnik Wiederholungsaufnahmen mit geringeren Abweichungen als bisher erstellen lassen, wobei eine Positioniermöglichkeit sowohl für den bezahnten als auch für den teilbezahnten oder zahnlosen Patienten gegeben sein soll.

Zur Lösung der gestellten Aufgabe wird erfindungsgemäß vorgeschlagen, ein Zähnärztliches Rontgendiagnostikgerät gemäß dem ersten Teil des Anspruchs 1 mit den Merkmalen gemäß dem zweiten Teil des Anspruchs 1 auszustatten. Für Aufnahmen am bezahnten Patienten besteht das Anlageteil vorteilhafterweise aus einem an sich bekannten Aufbißteil, auf das der Patient mit den Frontzähnen auf beißt. Im Gegensatz zu dem vorbekannten Gerät erfolgt die Positionierung des Patientenkopfes im Bereich unterhalb der Gesichtsmitte nicht mehr über eine Kinnauflage, sondern ausschließlich über das fest, in definierter Position am Träger gehalterte Anlageteil, gegen das bei einem bezahnten Patienten die Frontzähne und bei einem unbezahnten Patienten die Subnasale anliegt. Das Anlagereil ist sowohl Anlage- bzw. Fixierungspunkt als auch gleichzeitig Drehpunkt für den Patientenkopf beim Ausrichten auf die "Frankfurter Horizontale". Im Gegensatz zu dem vorbekannten Gerät, bei dem die Stirnstütze sowohl in der Horizontalen als auch in der Vertikalen verstellbar ist und auch die Kinnauflage mit dem dort schwenkbar gehalterten Aufbißstäbchen in der Horizontalen verstellt werden kann, ist nunmehr — abgesehen von Bißanomalien, bei denen eine Verstellung der Anlageteile (z.B. durch Verstellung der in der Beschreibung erwähnten Haltearme 12) notwendig ist — ausschließlich die Stirnstütze und diese nur in der Horizontalen verstellbar angeordnet. Das Positionieren des Patientenkopfes ist dadurch auch einfacher. Der patient wird zunächst subnasal oder frontal mit den Schneidezähnen am Anlageteil fixiert, anschließend, gegebenenfalls unter Zuhilfenahme eines Lichtvisiers, auf die "Frankfurter Horizontale" eingestellt; danach braucht lediglich die Stirnstütze in der Horizontalen so weit an den Patienten herangefahren zu werden, bis diese am Patientenkopf anliegt. Der in dieser Stellung angezeigte Wert ist für Repro-Aufnahmen festzuhalten.

Je nachdem, ob eine Standard-, eine Sinus- oder eine Kiefergelenk-Aufnahme erstellt werden soll, kann ein, den unterschiedlichen Aufnahmen, d.h. Schichtlagen-Positionen, gerechtwerdendes, in Längen- und Tiefenmaß unterschiedliches Anlageteil in den Träger eingesetzt werden. Alternativ kann auch der Träger um die entsprechenden Längen- und Tiefenmaße verstellbar angeordnet sein, so daß mit ein und demselben Anlageteil sowohl Standardals auch Sinus- bzw. Kiefergelenkaufnahmen erstellt werden können. Der Träger kann vorteilhafterweise eine exzentrisch gelagerte Walze od.dgl. sein, an dcrem Umfang mehrere Aufnahmeeinrichtungen in Form von Bohrungen, Zapfen, Schlitzen od.dgl. vorgesehen sind, wobei durch die exzentrische Anordnung der Anlagepunkt für die Frontzähne bzw. Subnasale entsprechend der oben erläuterten unterschiedlichen Schichtlagen-Position erzielt wird.

Für Kiefergelenkaufnahmen, bei denen man zur besseren Erkennung der Gelenkspalte einerseits eine Aufnahme bei geöffnetem Patientenmund (offener Biß) und andererseits bei geschlossenem Mund (Schlußbiß) erstellen will, ist es vorteilhaft, unterschiedliche, den Öffnungswinkel des Patientenmundes bestimmendc Aufbißteile vorzusehen, wobei es besonders vorteilhaft ist, wenn man an jenem Aufbißteil, mit welchem man Aufnahmen mit Schlußbiß erstellt, gleichsam ein den Öffnungswinkel für eine Aufnahme mit offenem Biß bestimmendes Abstandsteil anordnet, welches nach Erstellen der Aufnahme mit offenem Biß in die Position für die weitere Aufnahme mit Schlußbiß gebracht wird, ohne hierzu die Fixierung des Patienten ändern zu müssen. Es können also ohne Änderung der Patientenpositionierung beide Aufnahmen unmittelbar hintereinander erstellt werden. Das Abstandsteil kann ausschwenk- oder -klappbar am Anlageteil gehalten sein oder auch durch geeignete Führungsmittel, z.B. eine Schwalbenschwanzführung, längsverschiebbar gehalten sein.

Für zahnlose oder teilbezahnte Patienten erfolgt die Fixierung subnasal, d.h. unmittelbar unterhalb der Nase des Patienten. Einhierzu entsprechend ausgebildeter Anlagestab, der am Träger wiederum in definierter Stellung fest angeordnet ist, kann, wie bereits erläutert, für die unterschiedlichen Aufnahmearten (Standard-, Sinus-, Kiefergelenk-Aufnahmen) in Länge und Tiefe unterschiedlich gestaltet sein oder auf einer entsprechend verstellbar angeordneten Halterung befestigbar sein. Vorteilhaft ist es, die Halterung des Anlagestabes oberhalb der Zahnebene, z.B. an der Stirnstütze selbst, anzuordnen, wodurch Abbildungen des Stabes auf der Aufnahme vermieden werden.

Um reproduzierbare Aufnahmen erstellen zu können, bei denen die Okklusion zu überprüfen ist, wird vorgeschlagen, am Träger, an dem das Anlageteil, also beispielsweise ein Aufbißteil oder ein subnasaler Anlagestab, befestigbar ist, eine kombinierte Klapp-/Schwenkeinrichtung vorzusehen, die eine Aufbißplatte enthält, mit welcher zusammen mit der Stirnstütze eine Patientenkopffixierung, wie beschrieben, durchgeführt werden kann, wobei nach der Fixierung des Patientenkopfes die Aufbißplatte mittels der Schwenkeinrichtung aus dem Patientenmund und anschließend aus dem Strahlen- bzw. Belichtungsbereich herausgeschwenkt bzw. -geklappt werden kann, ohne daß der Oberkiefer dabei verstellt wird. Nach Entnahme der Aufbißplatte kann eine Aufnahme mit echtem Schlußbiß, also ohne Zwischenlage eines, wenn auch noch so dünnen Aufbißteiles, durchgeführt werden.

Weitere vorteilhafte Ausgestaltungen und Weiterbildungen der Erfindung sind in den Unteransprüchen enthalten. Mehrere Ausführungsbeispiele der Erfindung werden anhand der Zeichnung näher erläutert.

Es zeigen:

Figur 1 ein Röntgendiagnostikgerät nach der Erfindung in schaubildlicher Darstellung,

Figur 2 einen Ausschnitt aus dem Gerät nach Figur 1,

Figur 3 einen Teil des Gerätes nach Figur 2 in Seitenansicht in zwei verschiedenen Positionen,

Figuren 4 bis 8 für Kiefergelenkaufnahmen modifizierte Anlageteile,

Figuren 9 bis 11 eine weitere Modifikation eines Anlageteiles,

Figur 12 eine weitere Ausführungsform eines Anlageteiles,

Figur 13 eine Ausführungsform eines Anlageteiles für teilbezahnte bzw. zahnlose Patienten,

Figuren 14 bis 16 Einzelheiten der Halterung eines Anlageteiles,

Figuren 17 und 18 ein Anlageteil für Aufnahmen, die im Schlußbiß über die Okklusionsebene gemacht werden,

Figuren 19 und 20 zwei Varianten einer Halterung des Anlageteiles nach Figuren 17 und 18.

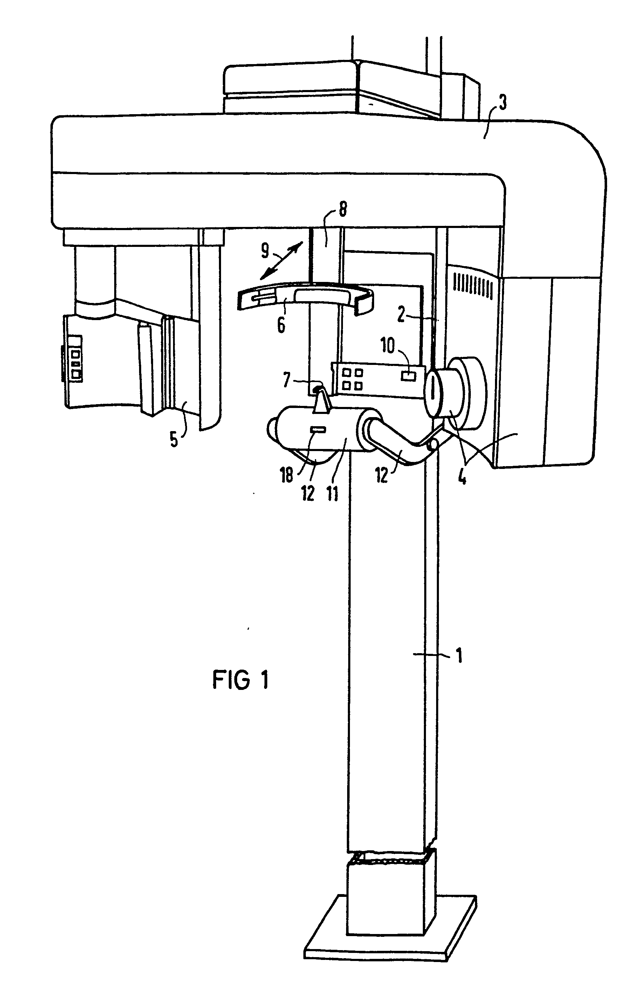

Figur 1 zeigt in einer schaubildlichen Darstellung ein zahnärztliches Röntgendiagnostikgerät zur Erstellung von Panorama-Schichtaufnahmen, enthaltend eine Säule 1, an der ein Laufwagen 2 in der Höhe verstellbar angeordnet ist, an dem eine Dreheinheit 3 befestigt ist, die in bekannter Weise eine Röntgenstrahlenquelle 4 und eine Filmkassette 5 trägt. Aufbau und Wirkungsweise dieser Teile sind hinreichend bekannt, brauchen deshalb an dieser Stelle nicht näher erläutert zu werden.

Zur Fixierung des Patientenkopfes ist einerseits eine Stirnstütze 6 und andererseits ein Anlageteil 7 vorgesehen. Die Stirnstütze 6 ist mittels eines vertikalen Trägers 8 an der Dreheinheit 3 befestigt und in Richtung des Pfeiles 9 in der Horizontalen verstellbar. Die eingestellte Position wird an einem Display 10 digital angezeigt.

Das Anlageteil 7 ist an einem zylindrischen Träger 11 abnehmbar befestigt, der wiederum an beidseitig angeordneten, mit dem Laufwagen 2 verbundenen Haltearmen 12 schwenkbar angeordnet ist, und zwar so, daß der Träger 11 in zwei definierte Raststellungen geschwenkt werden kann.

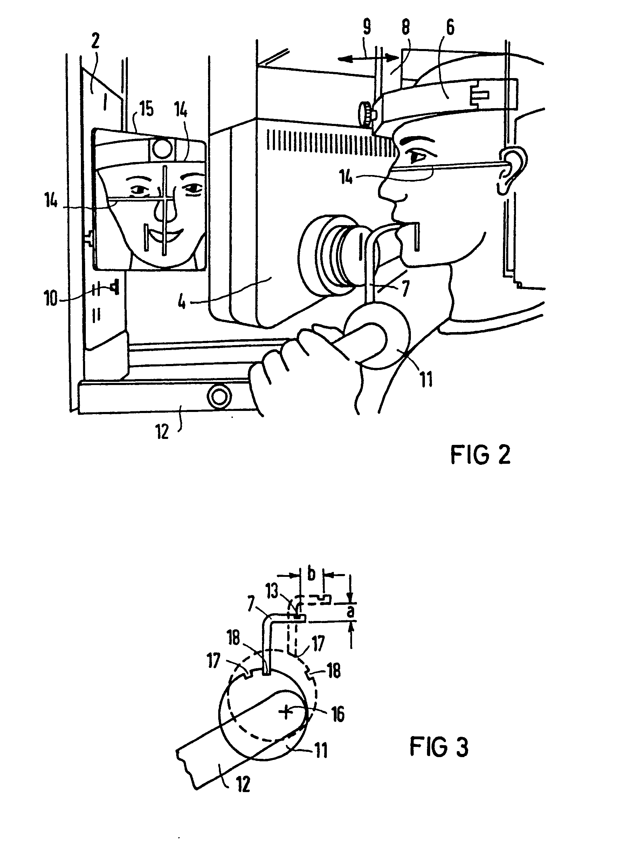

Die Figur 2 zeigt von einer anderen Perspektive aus gesehen einen Ausschnitt von dem Gerät mit positioniertem Patientenkopf. Aus der Darstellung ist zu entnehmen, daß das Anlageteil 7 ein zum Patientenmund hin gebogenes Teil ist. Es enthält am freien, in den Patientenmund einführbaren Ende eine Kerbe oder auch eine Erhebung, die eine Anlagefläche zur Anlage der Frontzähne des Patienten bildet. In Figur 3 ist diese Anlagefläche durch eine mit der Position 13 bezeichnete Kerbe gebildet.

Vor Fixierung des Patientenkopfes wird zunächst durch Verstellen des Laufwagens 2 die gesamte Dreheinheit und damit auch das Anlageteil 7 auf eine der Patientengröße entsprechende Höhenlage ausgerichtet. Danach bringt der Patient seine Frontzähne an der durch die Kerbe 13 gebildeten Anlagefläche zur Anlage. Der Patientenkopf wird nun mit Hilfe eines an sich bekannten Lichtvisiers, dessen Lichtstrahlen mit 14 bezeichnet sind, gegebenenfalls unter Zuhilfenahme eines Spiegels 15, in bezug auf die "Frankfurter Horizontale" sowie inbezug auf die Gesichtsmitte ausgerichtet. Die Kerbe 13, in die die Frontzähne eingreifen, bildet dabei einen Drehpunkt, um den der Patientenkopf so lange gedreht wird, bis er in bezug auf die "Frankfurter Horizontale" ausgerichtet ist. Nach Ausrichten des Kopfes wird die Stirnstütze 6 in Pfeilrichtung 9 so weit verstellt, bis diese am Patientenkopf fest zur Anlage kommt. Der am Anzeigedisplay 10 sich dabei ergebende Wert wird für eine spätere Repro-Aufnahme notiert.

Um neben Standard-Aufnahmen auch die eingangs bereits erläuterten Sinus- und Kiefergelenk-Aufnahmen machen zu können, sind entweder in Höhen- und Tiefenmaß unterschiedliche Anlageteile 7 vorzusehen oder es kann auch, wie anhand Figur 3 näher erläutert, ein und dasselbe Anlageteil vorgesehen werden, wenn der Träger um das entsprechende Höhen- und Tiefenmaß (a, b) verstellbar angeordnet ist. Infolge der unterschiedlichen Schichtebenen für einerseits Standard- und andererseits Sinus- bzw. Kiefergelenk-Aufnahmen muß das Anlageteil für Standardaufnahmen nämlich um etwa 20 bis 22 mm größer bemessen sein als das für Sinus- bzw. Kiefergelenkaufnahmen. Um diese Maßabweichungen zu kompensieren, ist der im wesentlichen als zylindrische Walze ausgebildete Träger 11 mittels einer Achslagerung 16 exzentrisch gelagert, wobei die exzentrische Lagerung dabei mit dem Durchmesser der Walze so abgestimmt ist, daß beim Einstecken des Anlageteils 7 in eine erste Aufnahmeöffnung 17 die erforderliche Position für Standard-Aufnahmen (gestrichelte Darstellung) und in eine zweite Aufnahmeöffnung 18 die Schichtlageposition für Sinus- und Kiefergelenk-Aufnahmen (durchgezogene Linien) erreicht werden.

Anstelle der Aufnahmeöffnungen 17 und 18 für das Anlageteil 7 können auch Schlitze, Zapfen u.dgl. vorgesehen sein; in jedem Falle ist jedoch sicherzustellen, daß das Anlageteil in einer genauen, durch Anschlag definierten und gegen Verdrehung gesicherten Position am Träger gehalten wird. Ein Ausführungsbeispiel einer solchen Halterung wird später noch näher erläutert.

Die exzentrisch gelagerte Walze ist, um eine exakte Position in den beiden Schwenkstellungen zu haben, mit einer nicht dargestellten Rasteinrichtung versehen. Sie kann außerdem, um Fehleinstellungen bzw. Fehlaufnahmen durch falsche Positionsaufnahmekombination zu vermeiden, mit einer elektrischen Kontaktsicherung ausgestattet sein.

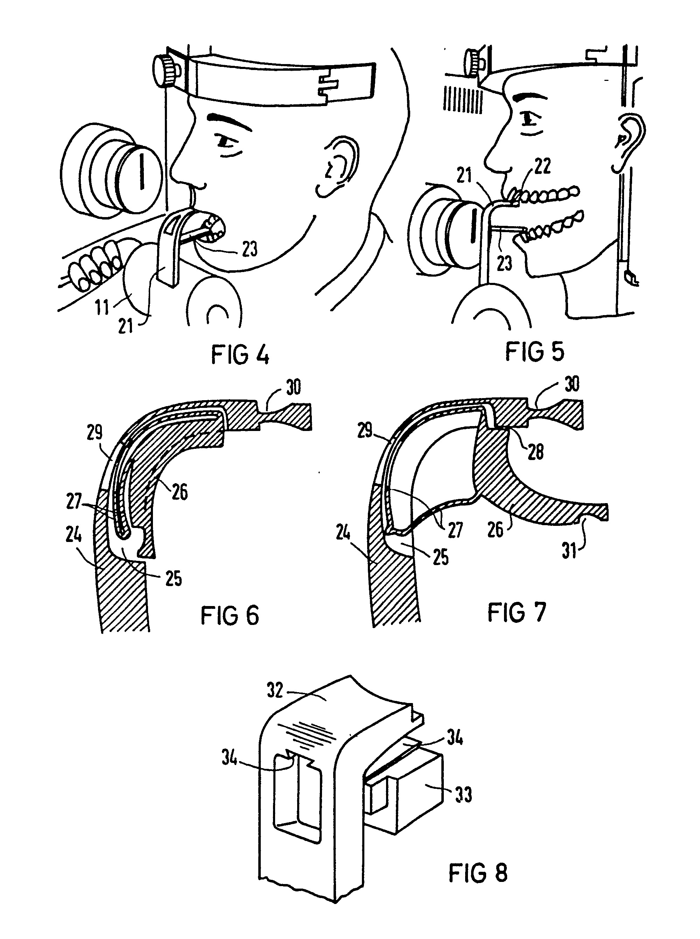

Die Figuren 4 bis 8 zeigen Ausführungsformen eines modifizierten Anlageteils, welche für Kiefergelenkaufnahmen geeignet sind. Bei Kiefergelenkaufnahmen erstellt man in der Regel zwei Aufnahmen, eine mit offenem Biß, also bei geöffnetem Patientenmund, und eine zweite Aufnahme mit Schlußbiß, d.h. bei geschlossenem patientenmund. Das hierzu im Träger 11 gehaltene Anlageteil 21 ist, ähnlich wie das Anlageteil 7, zum Patientenmund hin abgebogen, wobei das obere, in den Patientenmund einführbare Ende wiederum eine Kerbe 22 für die Anlage der vorderen Schneidezähne des Patienten enthält. Das Anlageteil 21 enthält ferner ein Abstandsglied 23, welches in einem definierten Abstand, der dem gewünschten Öffnungswinkel bei einer Aufnahme mit offenem Biß entspricht, aus einer Nichtgebrauchslage in eine Gebrauchslage ausgeklappt werden kann.

Die Figuren 6 und 7 zeigen eine besonders vorteilhafte Ausführung eines solchen Anlageteils, wobei die Figur 6 das Abstandsglied in der Nichtgebrauchs- und in Figur 7 in der Gebrauchslage zeigen. Das Anlageteil 24 enthält im Bereich der Krümmung eine Ausnehmung 25 zur Aufnahme eines der Krümmung etwa angepaßten Abstandsgliedes 26 sowie zweier ein Klappscharnier bildender Teile 27, die es ermöglichen, das Abstandsglied 26 aus der in Figur 6 gezeigten eingeklappten Position in die in Figur 7 dargestellte ausgeklappte Position zu bringen und in dieser Position auch rastend zu halten. Im ausgeklappten Zustand liegt das Abstandsglied 26 an der mit 28 bezeichneten Stelle am Anlageteil 24 an, so daß eine definierte Position gegeben ist. Das Ausklappen kann über eine im Bereich der Krümmung angeordnete Aussparung 29 vorgenommen werden. Eine die Auflagefläche für die Frontzähne bildende Kerbe ist für die Zähne des Oberkiefers mit 30, für die des Unterkiefers mit 31 bezeichnet.

Die Figur 8 zeigt eine Alternativlösung, bei der anstelle der ausschwenk- bzw. ausklappbaren Anordnung in einem Anlageteil 32 ein Abstandsglied 33 mittels einer Schwalbenschwanzführung 34 so gehalten ist, daß es in Richtung auf den Patienten hin innerhalb der Schwalbenschwanzführung verstellt werden kann, so daß einerseits eine Aufnahme mit offenem Biß und andererseits eine Aufnahme mit Schlußbiß, also ohne Abstandsglied, erstellt werden kann. Eine die Anlagefläche für die Frontzähne bestimmende Kerbe bzw. Erhebung ist hier wieder vorhanden.

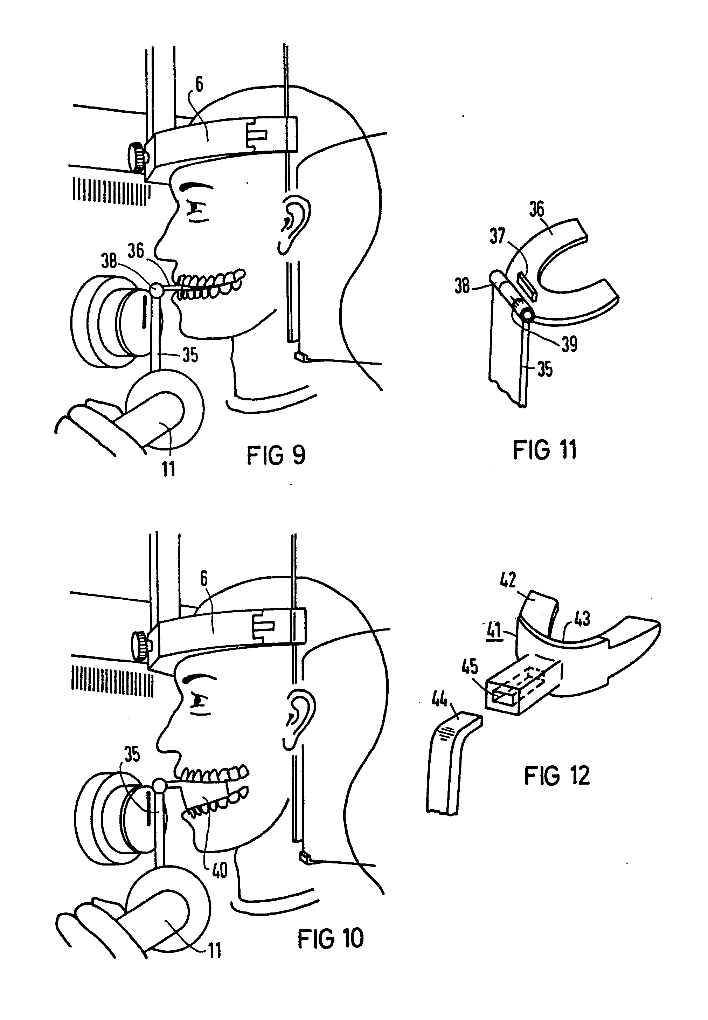

Die Figuren 9 bis 11 zeigen eine Ausführungsform, bei der ein Anlageteil 35 vorgesehen ist, dessen dem Patientenmund zugewandtes Ende 36 als Aufbißplatte (Figur 11) ausgebildet ist, die wiederum eine entsprechende, in Form einer Erhebung oder Vertiefung vorgesehene Anlagefläche 37 für die Frontzähne des Patienten enthält. Im Gegensatz zu den bisher beschriebenen Ausführungsformen erfolgt eine Positionierung hier über die Okklusionsebene, wozu die Aufbißplatte 36 mittels einer scharnierartigen Gelenkverbindung 38 mit dem übrigen Teil des Anlageteils 35 gelenkig verbunden ist. Außerdem ist zwischen den sich relativ zueinander bewegenden Teilen der Gelenkverbindung eine Markierung bzw. Skalierung 39 vorgesehen, die dazu dient, die Winkelposition zwischen Aufbißplatte und dem übrigen Teil des Anlageteils 35 für Repro-Aufnahmen feststellen zu können. Auch hier kann, wie in Figur 10 gezeigt, für Aufnahmen mit offenem Biß ein entsprechendes Abstandsglied 40 vorgesehen sein. Bei diesem Ausführungsbeispiel ist die Gelenkverbindung 38 so gestaltet, daß die Aufbißplatte 36 (Figur 9) leicht durch das Abstandsglied 40 (Figur 10) und umgekehrt ersetzt werden kann.

Die Figur 12 zeigt eine Ausführungsform, bei der ein Anlageteil 41, welches wiederum eine Aufbißplatte 42 sowie eine durch eine Erhebung 43 gebildete Anschlagfläche für die Frontzähne enthält, auf einen gekrümmten Stab 44 aufsetzbar ist, der, wie anhand des Anlageteils 7 erläutert, am Träger 11 in Figur 1 entsprechend gehaltert wird. Damit eine exakte definierte Position der Teile zueinander gegeben ist, weisen der gekrümmte Stab 44 an seinem freien Ende ein von der Kreisform abweichendes Profil und das Teil 41 eine entsprechend ausgebildete Aufnahmeöffnung 45 mit definiertem Tiefenanschlag auf. Bei dieser Variante führt der Patient zunächst das Anlageteil 41 in den Mund ein, beißt unter Beachtung des Schneidezahnanschlages fest zu und fädelt anschließend das Teil auf den gekrümmten Stab 44 bis zum Anschlag auf.

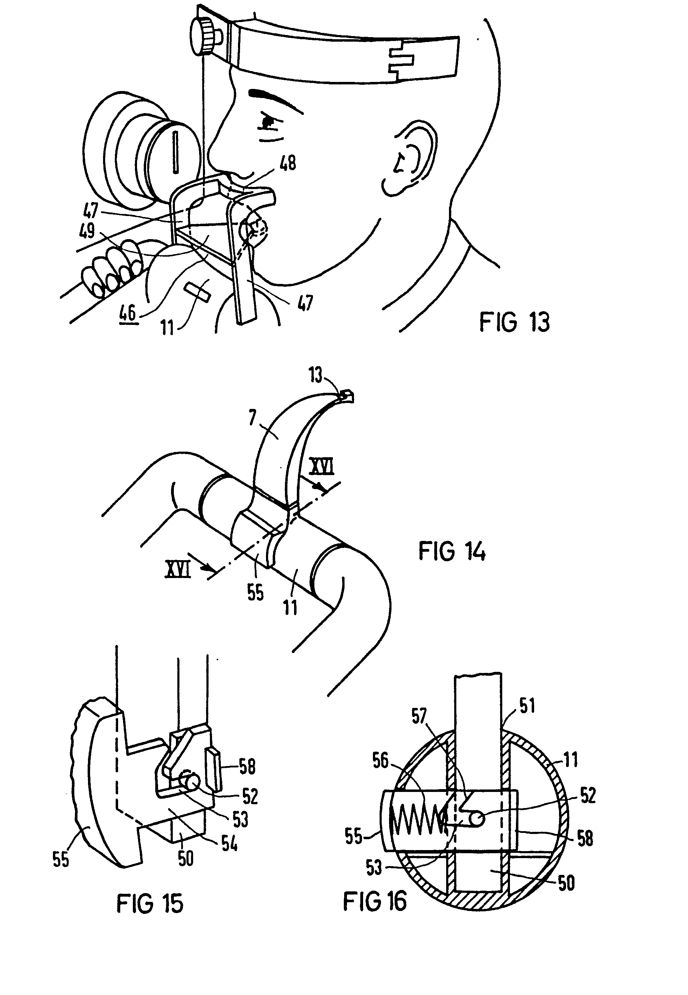

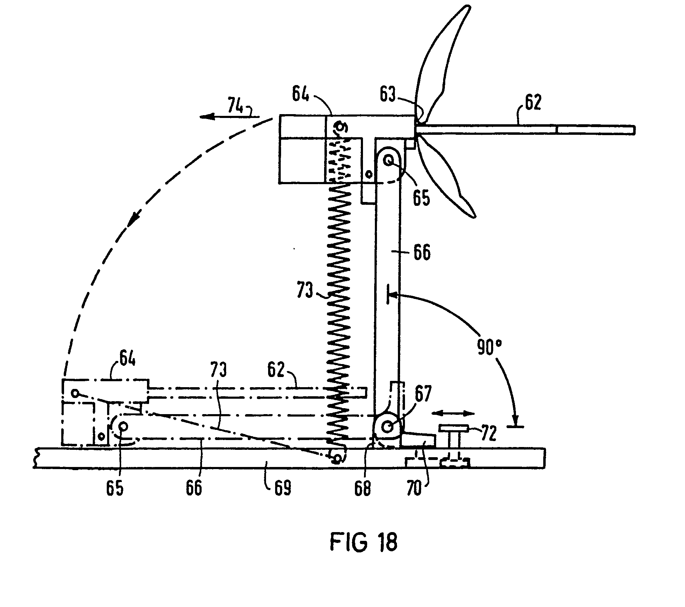

Die Figur 13 zeigt eine Ausführungsform eines Anlageteils 46, welches dazu geeignet ist, bei einem teilbezahnten bzw. zahnlosen Patienten als Positionierungsmittel vorgesehen zu werden. Das Anlageteil 46 ist hier bügelförmig ausgebildet, wobei die beiden Schenkel 47 entweder, wie für das Anlageteil 7 beschrieben, in entsprechende Aufnahmebohrungen am Träger 11 in definierter Lage einsetzbar sind oder seitlich des Trägers 11 in definierter Position gehaltert werden können. Der die Schenkel 47 verbindende Steg 48 ist so gestaltet, daß er eine subnasale, d.h. unmittelbar unterhalb der Nase des Patienten liegende, Anlagefläche für den Patienten bildet. Vorteilhafterweise kann dieser Verbindungssteg zumindest an der dem Patienten zugewandten Seite mit einer nicht kompressiblen Weichauflage versehen sein. Für Kiefergelenk-Aufnahmen kann auch hier wieder ein mit 49 bezeichnetes Abstandsglied vorgesehen sein, das bei Aufnahmen mit offenem Biß, wie erläutert, in eine entsprechende Gebrauchsstellung geschwenkt werden kann. Die bügelartige Halterung hat den Vorzug, daß bei der Aufnahme das Anlageteil nicht mit abgebildet wird.

Die Figuren 14 bis 16 zeigen am Beispiel des Anlageteiles 7 Einzelheiten einer Halterung des Anlageteiles am Träger 11.

Das dem Patientenmund abgewandte Ende 50 des Anlageteiles 7 enthält einen von der Kreisform abweichenden, z.B. rechteckigen, Querschnitt. Der Träger 11 enthält einen vom Öffnungsquerschnitt entsprechend abgestimmten Schacht 51, in den das Ende 50 verdrehsicher eingesteckt werden kann. Im eingesteckten Zustand greifen beidseitig des Anlageteils 7 angeordnete Rastzapfen 52 in Schlitze 53 ein, welche an seitlichen Stegen 54 eines Druckknopfes 55 vorhanden sind. Die Rastposition (Figuren 15 und 16) ist mittels einer Druckfeder 56 unterstützt. Bei Andruck auf den Druckknopf 55 wird die Rastverbindung, wie ersichtlich, gelöst. Eine Einlaufschräge 57 erleichtert das selbsttätige Einrasten beim Einschieben des Endteils 50 in den Schacht 51. Mit 58 ist ein Anschlag bezeichnet, der bei entnommenem Anlageteil an einer entsprechenden Wandung am Träger 11 anliegt und dadurch den Druckknopf gegen Herausfallen aus dem Träger sichert.

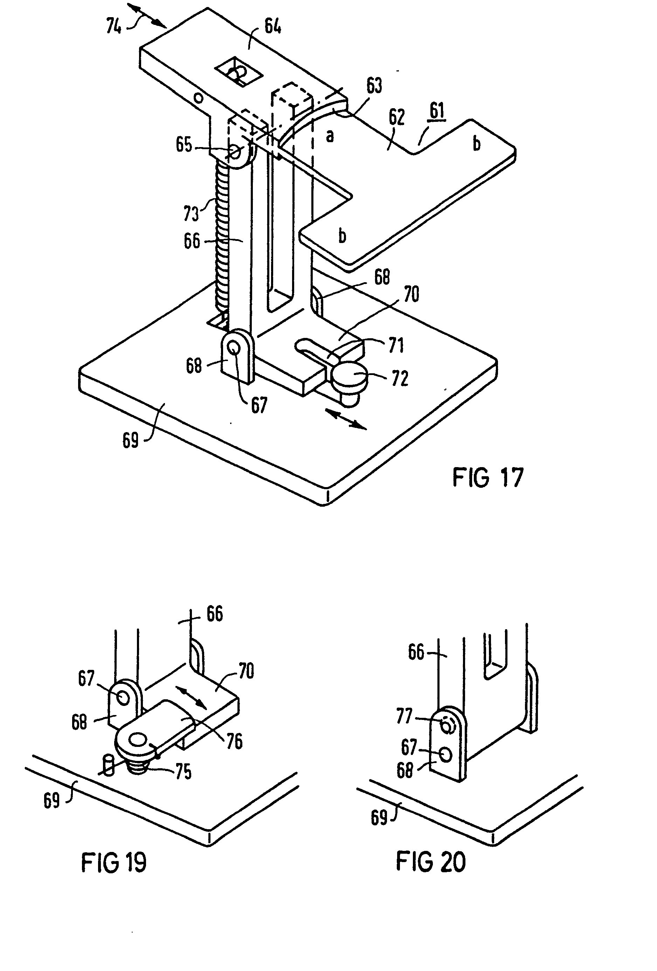

Die Figuren 17 und 18 zeigen einerseits in schaubildlicher Darstellung und andererseits in Seitenansicht eine Ausführungsform eines Anlageteils 61, welches dazu dient, reproduzierbare Aufnahmen mit echtem Schlußbiß, d.h. mit direkt aufeinanderliegenden Zähnen von Ober- und Unterkiefer, zu erstellen, und zwar so, daß zwischen der Positionierung mit Hilfe des Anlageteils und der Wegnahme des Anlageteils keine Positionsänderung des Patientenkopfes auftreten kann.

Das Anlageteil 61 enthält eine T-förmig ausgebildete, eine 3-Punkt-Auflage für die Zähne bildende Aufbißplatte 62, an deren mit a bezeichneten Stelle die Frontzähne und mit b bezeichneten Stellen wenigstens ein Molarzahn zur Anlage kommen. Auch hier bildet eine Erhebung 63 eine Anschlagfläche für die Frontzähne (Figur 18). Die Aufbißplatte 62 ist aus hygienischen Gründen in einer Halterung 64 leicht auswechselbar gehaltert. An der Halterung 64 ist mittels einer Gelenkverbindung 65 mit seinem einen Ende ein Gelenkarm 66 gehalten, dessen anderes Ende mittels einer Gelenkverbindung 67 an seitlichen Halteteilen 68 angelenkt ist. Diese Halteteile 68 sind mit einem dem Träger 11 in Figur 1 entsprechenden Träger 69 fest verbunden. Der Gelenkarm 66 enthält ein abgewinkeltes, leicht abgeschrägtes Fußende 70 mit einem Schlitz 71, in den ein im Träger 69 verschiebbar angeordneter Knopf 72 zur Festklemmung des Gelenkarmes einführbar ist. Mit 73 ist eine Zugfeder bezeichnet, die einerseits an der Halterung 64 und andererseits am Träger 69 angelenkt ist.

Die Funktion der Anordnung wird anhand Figur 18, die das Anlageteil 61 einerseits in Gebrauchsstellung und andererseits in (zusammengeklappter) Nichtgebrauchsstellung zeigt, erläutert.

In der Gebrauchsstellung die der Positionierung des Patientenkopfes dient, steht der Gelenkarm 66 in der in Figur 18 gezeigten vertikalen Stellung. In dieser position, in der der Gelenkarm mittels der Rastverbindung 71, 72 noch festgeklemmt ist, erfolgt die Positionierung des Patientenkopfes, wie schon geschildert, d.h. Aufbiß des Patienten auf die Aufbißplatte 62 mit Anlage der Frontzähne an der Erhebung 63 und Anlage des Kopfes an der Stirnstütze 6. Nachdem die Kopfpositionierung abgeschlossen ist, wird die Verriegelung 71, 72 gelöst. Der Patient öffnet nun leicht den Mund, wodurch sich der Unterkiefer von der Aufbißplatte leicht nach unten bewegt. Die Anordnung der Teile und der Feder gewährleisten, daß auch bei Lösen der Entriegelung 71, 72 die Afbißplatte in der dargestellten Positionierstellung, also mit lotrecht stehendem Gelenkarm 66, verbleibt. Wird nun durch einen leichten Zug an der Halterung 64 diese in Richtung des Pfeiles 74 bewegt, so klappt der Gelenkarm 65 um die Achse der Gelenkverbindung 67, wodurch die Aufbißplatte 62 aus dem Patientenmund und dabei gleichzeitig etwas nach unten bewegt wird; dabei wird der Oberkiefer nicht berührt, d.h. die zuvor eingestellte Position des Oberkiefers bleibt erhalten. Infolge der Federwirkung wird die Vorrichtung, wie in Figur 18 dargestellt, so weit zusammengeklappt, daß Gelenkarm und Aufbißplatte übereinander zu liegen kommen. Der Patient kannin dieser Stellung nun einen vollständigen Schlußbiß vollziehen und ist für eine Aufnahme in dieser Schlußbißstellung vorbereitet.

Die Befestigung des Gelenkarmes kann auf verschiedene Art und Weise erfolgen. Die Figuren 19 und 20 zeigen zwei Varianten. Bei dem einen Ausführungsbeispiel nach Figur 19 ist eine durch eine Feder 75 belastete Klemmscheibe 76 vorgesehen, die seitlich leicht weggeschwenkt werden kann; bei der Ausführung nach Figur 20 enthält der Gelenkarm 66 kein winkelig angesetztes Fußende, vielmehr ist dort zu beiden Seiten des Gelenkarmes eine Kugelrastung 77 vorgesehen, welche den Gelenkarm in der vertikalen Gebrauchsstellung des Anlageteils hält.

**Patentansprüche**

1. Zahnärztliches Röntendiagnostikgerät zur Erstellung kongruenter Panorama-Schichtaufnahmen vom Kiefer eines Patienten, enthaltend eine Dreheinheit (3) mit Strahlenquelle (4) und Filmkassette (5) und wenigstens zwei, den patientenkopf in bezug auf die Dreheinheit (3) festlegende Positionierglieder (6, 7), von denen das eine eine in der Horizontalen verstellbare Stirnstütze (6) ist, und enthaltend ferner Anzeigemittel (10) für zumindest die Verstellposition der Stirnstütze (6), dadurch gekennzeichnet, daß das andere Positionierglied ein an einem mit der Dreheinheit (3) verbundenen Träger (11) in definierter Stellung fest angeordnetes Anlageteil (7, 21, 24, 35, 41, 46, 61) ist, welches mit definierten Anschlagflächen (13, 22, 30, 31, 37, 43, 48) versehen ist, an denen bei Aufnahmen am bezahnten Patienten die Frontzähne und bei Aufnahmen am teilbezahnten bzw. zahnlosen Patienten die Subnasale des Patienten anliegen, wobei diese Anschlagflächen gleichsam eine horizontale Drehachse bilden, um die der Patientenkopf in bezug auf seine Gesichtssymmetrie (Frankfurter Horizontale) ausrichtbar ist.

2. Gerät nach Anspruch 1, dadurch gekennzeichnet, daß für Aufnahmen am bezahnten Patienten ein Träger (11) vorgesehen ist, in dem alternativ ein die Frontzähne fixierendes Anlageteil (7) für einerseits Standard- und andererseits Sinus- bzw. Kiefergelenk-Aufnahmen verdrehsicher rastend einsetzbar ist.

3. Gerät nach Anspruch 1, dadurch gekennzeichnet, daß das Anlageteil (21, 24, 32, 35, 46) mit einem den Patientenmund im geöffneten Zustand haltenden Abstandsteil (23, 26, 33, 40, 49) versehen ist.

4. Gerät nach Anspruch 3, dadurch gekennzeichnet, daß das Abstandsteil (23, 26, 33, 46) am Anlageteil (21, 24, 32) verstellbar derart angeordnet ist, daß es aus einer Nichtgebrauchsstellung, in der das Anlageteil für Aufnahmen mit geschlossenem Patientenmund dient, in eine Gebrauchsstellung für Aufnahmen mit geöffnetem Patientenmund bringbar ist.

5. Gerät nach Anspruch 4, dadurch gekennzeichnet, daß das Abstandsteil (26) in der Nichtgebrauchslage in einer Ausnehmung (25) des Anlageteils (24) angeordnet ist und mittels Gelenkverbindung (27) aus dieser Nichtgebrauchsstellung in die für eine Aufnahme mit geöffnetem Patientenmund geeignete Gebrauchsstellung schwenkbar ist.

6. Gerät nach Anspruch 5, gekennzeichnet durch ein zum Patientenmund hin gekrümmtes, an seinem freien Ende eine Anlage für die Frontzähne des Oberkiefers aufweisendes Teil (24), in dessen Krümmungsbereich das am freien Ende ebenfalls mit einer Anlage für die Frontzähne des Unterkiefers enthaltendes Abstandsteil (26) scharnierartig aus- und einklappbar und im ausgeklappten Zustand gegen Anschlagflächen (28) in definierter Position gehalten, angeordnet ist.

7. Gerät nach Anspruch 6, dadurch gekennzeichnet, daß im Bereich der Krümmung eine Ausnehmung (29) zur Betätigung des Schwenkmechanismus vorhanden ist.

8. Gerät nach Anspruch 3, dadurch gekennzeichnet, daß das Abstandsteil (33) mittels einer mit Führungsmitteln (34) versehenen Steckverbindung am Anlageteil (32) gehaltert ist.

9. Gerät nach Anspruch 1, dadurch gekennzeichnet, daß für Aufnahmen am teilbezahnten bzw. zahnlosen Patienten ein bügelförmiges Anlageteil (46) vorgesehen ist, dessen Schenkel (47) im Träger (11) in definierter Stellung gehalten sind und dessen Verbindungssteg (48) der Mundform im subnasalen Bereich angepaßt die Anlagefläche bildet.

10. Gerät nach Anspruch 1, dadurch gekennzeichnet, daß eine mit einer Anlagefläche für Frontzähne versehene Aufbißplatte (36, 62) vorgesehen ist, die so gestaltet ist, daß auf ihr in Dreipunktauflage die Frontzähne und wenigstens ein Molarzahn zu beiden Seiten sind.

11. Gerät nach Anspruch 10, dadurch gekennzeichnet, daß die Aufbißplatte (36, 62) an einem am Träger (11) befestigbaren Arm (35, 66) mittels eines Gelenks (38, 65, 67) mit horizontaler Achslagerung schwenkbar gehalten ist und an der Gelenkverbindung (38) eine die Schwenkstellung anzeigende Markierung (39) vorgesehen ist.

12. Gerät nach Anspruch 10, dadurch gekennzeichnet, daß die Aufbißplatte (62) an einer Halterung (64), vorzugsweise leicht lösbar, befestigt ist, die Teil einer Klapp-Schwenkvorrichtung (64, 65, 66, 73) ist und einen Gelenkarm (66) enthält, der mit seinem einen Ende mittels einer Klemmeinrichtung (71, 72, 75, 76, 77) in einer vertikal ausgerichteten Position mit dem Träger (11, 69) verbindbar ist und an dessen anderem Ende mittels eines weiteren Gelenks (65) die Halterun (64) für die Aufbißplatte (62) angelenkt ist, und daß zwischen Träger (11, 69) und Halterung (64) ein Kraftelement (73) derart angeordnet ist, daß der Gelenkarm (66) in der vertikalen Position stabil gehalten ist und bei geringfügigem Schwenken des Gelenkarms um die untere Gelenkachse (67) die Halterung (64) mit der Aufbißplatte (62) um diese Gelenkachse (67) in eine waagrechte Stellung geschwenkt wird, in welcher Gelenkarm (66) und Halterung (64) mit Aufbißplatte (62) übereinanderliegend angeordnet sind.

13. Gerät nach Anspruch 12, dadurch gekennzeichnet, daß das untere Ende des Gelenkarms (66) mittels rasch lösbarer Klemmeinrichtung (71, 72, 75, 76, 77) in der Vertikalstellung feststellbar ist und der Gelenkarm (66) mit einem abgewinkelten Fußende (70) versehen ist, an dem die Klemmeinrichtung (71, 72, 75, 76) angreift.

14. Gerät nach einem der Ansprüche 1 bis 13, dadurch gekennzeichnet, daß ein an einem mit der Dreheinheit (3) verbundenen Haltearm (12) exzentrisch gelagerter Träger (11) vorgesehen ist, der am Umfang mehrere, den unterschiedlichen Schichtlagen bzw. Positionen bei Standard-, Sinus- und Kiefergelenk-Aufnahmen berücksichtigende Aufnahmeeinrichtungen (17, 18, 51) zur festen Halterung eines für die unterschiedlichen Aufnahmearten gleichen Anlageteils (7) enthält.

15. Gerät nach Anspruch 14, dadurch gekennzeichnet, daß als Träger eine exzentrisch gelagerte und in den unterschiedlichen Aufnahmearten entsprechenden Stellungen arretierbare Walze (11) vorgesehen ist.

16. Gerät nach einem der Ansprüche 1 bis 15, dadurch gekennzeichnet, daß das Anlageteil (7, 21, 24, 32, 35, 41, 61) mittels einer druckknopfartig entriegelbaren Rastung (Figuren 14 bis 1) im Täge (11) in definierter Stellung verdrehsicher gehalten ist.

17. Gerät nach Anspruch 16, dadurch gekennzeichnet, daß das Anlageteil (7, 21, 24, 32, 35, 41, 61) an seinem dem Träger (11) zugewandten Ende (50) eine von der Kreisform abweichende Querschnittsform enthält und der Träger (11) einen dazu passenden Schacht (51) aufweist, in den das Ende (50) des Anlageteils einsteckbar ist, daß das Einsteckende (50) Verriegelungszapfen (52) aufweist, die in Rastschlitze (53) einer federbelastet am Träger (11) gehalterten Drucktaste (55) eingreifen und das Anlageteil im Träger rastend halten und daß die Drucktaste (55) mit seitlichen, die Rastschlitze (53) aufweisenden Stegen (54) versehen ist, die einerseits eine Einlaufschräge (57) für das selbsttätige Einrasten der Verriegelungszapfen (52) und andererseits Anschlagelemente (58) für eine Halterung der Drucktaste (55) in einer beinicht eingeführtem Anlageteil entsprechenden Position aufweisen.

## Revendications

1. Appareil de radiodiagnostic dentaire servant à établir des tomographies panoramiques congruentes de la mâchoire d'un patient, et contenant une unité rotative (3) comportant une source de rayonnement (4) et une cassette pour film (5) et au moins deux organes de positionnement (6, 7), qui fixent la tête du patient par rapport à l'unité rotative (3) et dont l'un est un appui frontal (6) réglable horizontalement, et comportant, en outre, des moyens d'enfichage (10) servant à indiquer au moins la position de réglage de l'appui frontal (6), caractérisé par le fait que l'autre organe de positionnement est un élément d'application (7, 21, 24, 35, 41, 46, 61), qui est monté fixe dans une position définie sur un support (11) relié à l'unité rotative (3) et comporte des surfaces de butée définies (13, 22, 30, 31, 37, 43, 48), contre lesquelles s'appliquent les dents de devant, dans le cas d'un patient ayant ses dents, et les dents sous-nasales du patient dans le cas de radiographies réalisées avec un patient ayant une partie de ses dents ou n'ayant plus ses dents, ses surfaces de butée formant pour ainsi dire un axe horizontal de rotation, autour duquel la tête du patient peut être orientée par rapport à sa symétrie faciale (plan horizontal de Francfort).

2. Appareil suivant la revendication 1, caractérisé par le fait que pour des radiographies effectuées sur des patients ayant leurs dents, il est prévu un support (11), dans lequel on peut insérer, de manière qu'il soit encliqueté avec blocage en rotation, alternativement un élément ou organe d'application (7) fixant les dents de devant d'une part pour des radiographies standards et d, autre part pour des radiographies des sinus et de l'articulation temporo-maxillaire.

3. Appareil suivant la revendication 1, caractérisé par le fait que l'élément d'application (21, 24, 32, 35, 46) comporte une pièce d'écartement (23, 26, 33, 40, 49) maintenant ouverte la bouche du patient.

4. Appareil suivant la revendication 3, caractérisé par le fait que la pièce d'écartement (23, 26, 33, 46) est disposée sur l'élément d'application (21, 24, 32) de manière à être réglable de telle sorte qu'elle peut être ramenée d'une position de non utilisation, dans laquelle l'élément d'application est utilisé pour des radiographies réalisées lorsque la bouche du patient est fermée, dans une position d'utilisation pour des radiographies effectuées lorsque la bouche du patient est ouverte.

5. Appareil suivant la revendication 4, caractérisé par le fait que, dans sa position de non utilisation, la pièce d'écartement (26) est disposée dans un évidement (25) de l'élément d'application (24) et peut être amenée par pivotement, au moyen d'une liaison articulée (27), depuis cette position de non utilisation dans la position d'utilisation convenant pour réaliser une radiographie lorsque le patient a la bouche ouverte.

6. Appareil suivant la revendication 5, caractérisé par une partie (24), qui est cintrée en direction de la bouche du patient et possède, sur son extrémité libre, une zone d'application pour les dents de devant de la mâchoire supérieure et dans la zone cintrée de laquelle la pièce d'écartement (26), qui comporte également sur son extrémité libre une zone d'application pour les dents de devant de la mâchoire inférieure, est montée de manière à pouvoir être ressortie et rétractée par rabattement à la manière d'une charnière et est maintenue à l'état ressorti par rabattement, dans une position définie, contre des surfaces de butée (28).

7. Appareil suivant la revendication 6, caractérisé par le fait qu'un évidement (29) prévu pour l'actionnement du mécanisme de pivotement est présent au voisinage de la zone cintrée.

8. Appareil suivant la revendication 3, caractérisé par le fait que la pièce d'écartement (33) est maintenue sur l'élément d'application (32) à l'aide d'une liaison enfichable pourvue de moyens de guidage (34).

9. Appareil suivant la revendication 1, caractérisé par le fait que pour des radiographies d'un patient ayant une partie de ses dents ou n'ayant plus ses dents, il est prévu un élément d'application en forme d'étrier (46), dont les branches (47) sont maintenues dans une position définie dans le support (11) et dont la barrette de liaison (48) forme la surface d'application qui est adaptée à la forme de la bouche dans la zone sous-nasale.

10. Appareil suivant la revendication 1, caractérisé par le fait qu'il est prévu une plaque à mordre (36, 62), qui comporte une surface d'application pour les dents de devant et est agencée de telle sorte que les dents de devant et au moins une molaire des deux côtés s'appliquent sur cette plaque conformément à un appui en trois points.

11. Appareil suivant la revendication 10, caractérisé par le fait que la plaque à mordre (36, 62) est maintenue sur un bras (35, 66) pouvant être fixé sur le support (11), à l'aide d'une articulation (38, 65, 67) de manière à pouvoir pivoter avec un tourillonnement horizontal d'axe et qu'une marque (39) indiquant la position de pivotement est prévue sur la liaison articulée (38).

12. Appareil suivant la revendication 10, caractérisé par le fait que la plaque à mordre (62) est fixée, de préférence de manière à être aisément amovible, sur un dispositif de retenue (64), qui fait partie d'un dispositif de rabattement/ pivotement (64, 65, 66, 73) et contient un bras articulé (66) qui peut être relié, par l'une de ses extrémités, au moyen d'un dispositif de serrage (71, 72, 75, 76, 77), au support (11, 69), dans une position verticale et sur l'autre extrémité duquel le dispositif de retenue (64) pour la plaque à mordre (62) est articulé à l'aide d'une autre articulation (65), et qu'un élément de transmission de force (73) est disposé entre le support (11, 69) et le dispositif de retenue (64) de telle sorte que le bras articulé (66) est maintenu stable en position verticale et, lors d'un très faible pivotement du bras articulé autour de l'axe inférieur d'articulation (67), le dispositif de retenue (64) muni de la plaque à mordre (62) est amené par pivotement, autour de cet axe d'articulation (67), dans une position horizontale, dans laquelle le bras articulé (66) et le dispositif de retenue (64) équipé de la plaque à mordre (62) sont disposés l'un au-dessus de l'autre.

13. Appareil suivant la revendication 12, caractérisé par le fait que l'extrémité inférieure du bras articulé (66) peut être fixée en position verticale à l'aide d'un dispositif de serrage (71, 72, 75, 76, 77) pouvant être libéré rapidement et que le bord articulé (66) comporte une extrémité inférieure coudée (70), à laquelle est accrochée le dispositif de serrage (71, 72, 75, 76).

14. Appareil suivant l'une des revendications 1 à 13, caractérisé par le fait qu'il est prévu un support (11), qui est monté de façon excentrée sur un bras de retenue (12) relié à l'unité rotative (3) et contient, sur son pourtour, plusieurs dispositifs en forme de renfoncements (17, 18, 51) qui tiennent compte des différentes attitudes ou position de couche lors de radiographies standards, de radiographies des sinus et de radiographies de l'articulation temporo-maxillaire, pour le maintien ferme d'un élément d'application (7) identique pour les différents types de radiographies.

15. Appareil suivant la revendication 14, caractérisé par le fait qu'il est prévu, comme support, un cylindre (11) monté excentré et pouvant être bloqué dans des positions correspondant aux différents types de radiographies.

16. Appareil suivant l'une des revendications 1 à 15, caractérisé par le fait que l'élément d'application (7, 21, 24, 32, 35, 41, 61) est maintenu d'une manière bloquée en rotation dans une position définie dans le support (11), à l'aide d'un encliquetage (figures 14 à 16) pouvant être déverrouillé à la manière d'un bouton-pression.

17. Appareil suivant la revendication 16, caractérisé par le fait que l'élément d'application (7, 21, 24, 32, 35, 41, 61) contient, sur son extrémité (50) tournée vers le support (11), une section transversale dont la forme diffère de la forme circulaire et que le support (11) comporte un renfoncement (51) adapté au support, dans lequel peut être enfichée l'extrémité (50) de l'élément d'application, que l'extrémité enfichable (50) comporte des tétons de verrouillage (52), qui s'engagent dans des fentes d'encliquetage (53) d'un bouton-poussoir (55) maintenu sur le support (11) en étant chargé par un ressort, et retiennent l'élément d'application à l'état encliqueté dans le support, et que le bouton-poussoir (55) comporte des barrettes latérales (54), qui possèdent les fentes d'encliquetage (53) et qui comportent d'une part un biseau d'entrée (57) pour l'encliquetage automatique des tétons de verrouillage (52) et d'autre part des éléments de butée (58) pour le maintien du bouton-poussoir (55) dans une position correspondante obtenue dans le cas où l'élément d'application n'est pas inséré.

## Claims

1. A dental x-ray diagnostic apparatus for creating congruent panoramic planigraphic exposures of the jaw of a patient, containing a rotary unit (3) with a radiation source (4) and film cassette (5) and at least two positioning members (6, 7) for adjusting the patient's head relative to the rotary unit (3), one of which is a forehead support (6) adjustable in the horizontal direction and also containing indicator means (10) for at least the adjusting position of the forehead support (6), characterised in that the other positioning member is a constructional part (7, 21, 24, 35, 41, 46, 61) arranged firmly in a defined position on a support (11) connected to the rotary unit (3), which part is provided with defined stop faces (13, 22, 30, 31, 37, 43, 48), on which the front teeth rest for exposures of the toothed patient, and on which the subnasal part of the patient rests for exposures of the partially toothed or toothless patient, with these stop faces also forming a horizontal rotary axis, around which the patient's head is able to be aligned relative to its facial symmetry (Frankfurter Horizontal).

2. An apparatus according to claim 1, characterised in that, for exposures of the toothed patient, a support (11) is provided in which there can be inserted in a rotatably-secure locking manner alternatively a constructional part (7) for fixing the front teeth, for standard exposures on the one hand and, for sinus or maxillary joint exposures on the other hand.

3. An apparatus according to claim 1, characterised in that the constructional part (21, 24, 32, 35, 46) is provided with a spacing member (23, 26, 33, 40, 49) for holding the patient's mouth in the open state.

4. An apparatus according to claim 3, characterised in that the spacing member (23, 26, 33, 46) is arranged on the constructional part (21, 24, 32) in an adjustable manner, so that it can be brought from a non-operating position in which the constructional part serves for exposures with the patient's mouth closed into an operating position for exposures with the patient's mouth open.

5. An apparatus according to claim 4, characterised in that, in the non-operating position the spacing member (26) is arranged in a recess (25) of the constructional part (24) and by means of a hinge joint (27) is able to pivot from this non-operating position into the suitable operating position for an exposure with the patient's mouth open.

6. An apparatus according to claim 5, characterised in that there is arranged a part (24) curved towards the patient's mouth, having a support for the front teeth of the upper jaw on its free end, in whose curved region there is arranged the spacing member (26), also containing a support for the front teeth of the lower jaw on its free end, able to be unfolded and folded like a hinge, and held in the unfolded state against stop faces (28) in a defined position.

7. An apparatus according to claim 6, characterised in that in the region of the curve a recess (29) is present for activating the pivoting mechanism.

8. An apparatus according to claim 3, characterised in that the spacing member (33) is held by means of a plug connection provided with guiding means (34) on the constructional part (32).

9. An apparatus according to claim 1, characterised in that, for exposures of the partially toothed or toothless patient, a bracket-shaped constructional part (46) is provided, the side piece (47) of which is held in the support (11) in a defined position and the connecting bar (48) of which forms the constructional surface adapted to the mouth shape in the subnasal region.

10. An apparatus according to claim 1, characterised in that a bite-down plate (36, 62) is provided which has a contact surface for the front teeth and is shaped so that the front teeth and at least one molar tooth are on both sides thereof in a three point contact.

11. An apparatus according to claim 10, characterised in that the bite-down plate (36, 62) is pivotably mounted on an arm (35, 66), which is securable to the support (11), by means of a hinge (38, 65, 67) having a horizontal axle bearing, and a mark (39) indicating the pivoting position is provided on the hinging joint (38).

12. An apparatus according to claim 10, characterised in that the bite-down plate (62) is secured on a holder (64), preferably easily releasable, which holder is part of a hinge/pivoting device (64, 65, 66, 73) and contains a hinge arm (66), which is able to be connected at one end to the support (11, 69) by means of a clamping device (71, 72, 75, 76, 77) in a vertically aligned position, and at the other end of which the holder (64) for the bite-down plate (62) is hinged by means of a further joint (65) and in that between the support (11, 69) and holder (64) a force element (73) is arranged so that the hinged arm (66) is held stably in the vertical position and, with slight pivoting of the hinged arm round the lower joint axis (67), the holder (64) with the bite-down plate (62) is pivoted around the joint axis (67) into a horizontal position in which the hinged arm (66) and the holder (64) are arranged with the bite-down plate (62) lying over each other.

13. An apparatus according to claim 12, characterised in that the lower end of the hinged arm (66) is able to be fixed in the vertical position by means of a quick release clamping device (71, 72, 75, 76, 77) and the hinged arm (66) is provided with an angled foot end (70), on which the clamping device (71, 72, 75, 76) engages.

14. An apparatus according to one of claims 1 to 13, characterised in that eccentrically mounted on the holding arm (12) connected to the rotary unit (3) is provided a support (11) which contains on the circumference, several accepting devices (17, 18, 51) for a secure hold of the same constructional part (7) for all the varying types of exposure, considering the different layers or positions with standard, sinus and maxillary joint exposures.

15. An apparatus according to claim 14, characterised in that an eccentrically mounted drum (11), able to be stopped in positions corresponding to various types of exposures is provided as support.

16. An apparatus according to one of claims 1 to 15, characterised in that the constructional part (7, 21, 24, 32, 35, 41, 61) is mounted in a defined position in the support (11) so as to be secure against rotation by means of a push-button type unlockable locking (Figures 14 to 16).

17. An apparatus according to claim 16, characterised in that the constructional part (7, 21, 24, 32, 35, 41, 61) contains on its end (50) facing the support (11) a cross-section deviating from the circular shape and the support (11) has a shaft (51) adapted thereto, in which the end (50) of the constructional part is able to be inserted, in that the inserting end (50) has locking plugs (52), which engage in a locking slot (53) of a push-button mounted in a spring-loaded manner on the support (11) and which hold the constructional part in a locking manner in the support and in that the push-button (55) is provided with lateral bars (54) having locking slots (53), which bars have, on the one hand, an entry bevel (57) for the automatic locking of the locking plug (52) and on the other hand stop elements (58) for holding the push-button (55) in a corresponding position when the constructional part is not introduced.

FIG 1

FIG 2

FIG 3

2

FIG 4

FIG 5

FIG 6

FIG 7

FIG 8

FIG 9

FIG 11

FIG 10

FIG 12

FIG 13

FIG 14

FIG 15

FIG 16

FIG 18

FIG 17

FIG 19

FIG 20